# EUROPEAN PATENT APPLICATION

(11) **EP 1 666 646 A1**
(43) Date of publication of application: **07.06.2006**
(21) Application number: 04771686.5
(22) Date of filing: 10.08.2004
(51) Int. Cl.: C30B 29/58, C07K 1/30

(54) **PROTEIN CRYSTALLIZATION APPARATUS, METHOD OF PROTEIN CRYSTALLIZATION, PROTEIN CRYSTALLIZING AGENT AND PROCESS FOR PREPARING THE SAME**

(30) Priority: 11.08.2003 JP 2003291308; 11.08.2003 JP 2003291433
(71) Applicant: MITSUBISHI RAYON CO., LTD., Tokyo 108-8506 (JP)
(72) Inventor: TANAKA, Isao, Sapporo-shi, Hokkaido 006-0812 (JP); WATANABE, Nobuhisa, Sapporo-shi, Hokkaido 065-0028 (JP); TAKEUCHI, Hiroshi, c/o Mitsubishi Rayon Co., Ltd., Yokohama-shi, Kanagawa 230-0053 (JP); ISEKI, Takayuki,c/o Mitsubishi Rayon Co., Ltd., Yokohama-shi, Kanagawa 230-0053 (JP); SHIBUYA, Nozomu,c/o Mitsubishi Rayon Co., Ltd., Yokohama-shi, Kanagawa 230-0053 (JP); NISHIJIMA, Chiharu,, Kawasaki-ku,Kawasaki-shi Kanagawa-ken (JP)
(74) Representative: HOFFMANN EITLE
(86) International application number: PCT/JP2004/011725
(87) International publication number: WO 2005/014899

(57) **Abstract**

The invention provides a protein crystallizing device capable of performing a protein crystallization experiment or screening of crystallization conditions rapidly and economically with a high reliability, and a protein crystallizing agent capable of performing the operation of protein crystallization by a simpler method.

The protein crystallizing device of the present invention comprises a protein crystallizing microarray having at least two crystallizing agent holding parts which hold a protein crystallizing agent and a plate laminated on the protein crystallizing microarray, wherein the plate has crystallizing sections corresponding to the crystallizing agent holding parts and capable of being filled with a protein-containing sample and recessed parts provided between the crystallizing sections. The protein crystallizing agent of the present invention is evenly held in a gel by gelatinizing a solution containing a protein precipitant and an unsaturated monomer.

## Description

### TECHNICAL FIELD

The present invention relates to a device and a method for performing crystallization of a protein or screening of crystallization conditions, and a protein crystallizing agent for precipitating protein crystals from a protein-containing sample, and a preparation method thereof.

### BACKGROUND ART

Recently, a study called the structural genome science has been carried out which attempts to clarify the relation between the steric structure of a protein and the function of the protein so as to elucidate the function of the gene encoding the protein.

In the analysis of the steric structure of a protein, normally the screening of the crystallization conditions of a protein to be analyzed is firstly performed. Then, crystallization is performed in the optimum crystallization conditions. By providing the obtained crystals to X-ray structure analysis, the steric structure of the protein is analyzed. Here, in the process of screening the crystallization conditions of a protein, a lot of time and a large amount of protein sample are spent until the conditions for obtaining excellent crystals enough to perform the steric structure analysis are determined. Therefore, it becomes a bottleneck in the steric structure analysis. Moreover, as well as the vapor diffusion method, various crystallizing methods have been contrived. However, there are still a lot of problems such as the complexity of the experimental procedure. New crystallizing methods replacing these existing methods and devices thereof have been developed, and there are proposed various protein crystallizing devices, crystallizing methods, and crystallization condition screening methods, for performing the screening of protein crystallization conditions, or performing crystallization rapidly with less amount of sample. For example, in Japanese Unexamined Patent Application, First Publication No. H06-321700, there is proposed a method in which a precipitant and a protein are contained in gels, which are then laminated, so as to grow crystals in the gels while suppressing the convection seen in a solution. Moreover, besides, in order to simplify the crystallizing method, an attempt has been made to use a gel-like material. Furthermore, various precipitants have been proposed for searching the crystallization conditions.

However, in the case of using a gel-like material, depending on the combination of the gel-like material and the precipitant, there have been problems in that the gel becomes opaque or that the gelatinization reaction does not progress. If the gel becomes opaque, the presence/absence of the crystallization state can not be observed by a microscope, causing a problem.

Moreover, some of the present inventors have proposed a crystallizing device which performs a crystallizing experiment on a microarray type chip, with an object of performing the screening of the crystallization conditions of a protein rapidly and economically with a minute amount of sample (for example, WO03/053998 pamphlet).

In the invention described in WO03/053998 pamphlet, a microarray is used. The microarray, has gel-like materials held in respective sections formed by through holes. The respective gel-like materials contain a plurality of types of and concentrations of protein crystallizing agents. By bringing this microarray into contact with the protein-containing sample, the screening of a plurality of crystallization conditions can be performed at once. Furthermore, it is very efficient since the screening can be performed with a minute amount of protein sample.

However, in the protein crystallizing device, the protein-containing samples and/or the crystallizing agents are moved and mixed (contaminated) between the sections in the microarray. Therefore, there has been concern in that the condition under which crystals were precipitated does not match the concentration and the type of a crystallizing agent previously contained in the gel-like material in the section.

Moreover, in such a device, it is necessary to manually supply a protein-containing sample to the crystallizing agent holding parts, which complicates the operation. Moreover, an automatic device which automatically supplies a protein-containing sample is expensive.

The present invention is made in order to solve the above problems. An object of the present invention is to provide a protein crystallizing agent for progressing the gelatinization reaction without making the gel opaque, and a preparation method thereof, and a protein crystallizing device capable of performing a protein crystallization experiment or screening of crystallization conditions rapidly and economically with a high reliability.

### DISCLOSURE OF INVENTION

The present invention provides the following:
- a protein crystallizing device comprising:
   a protein crystallizing microarray having at least two crystallizing agent holding parts which hold a protein crystallizing agent, and
   a plate laminated on said protein crystallizing microarray, said plate having
      crystallizing sections corresponding to said crystallizing agent holding parts so that the sections being capable of being filled with a protein-containing sample, and
      recessed parts provided between the crystallizing sections;
- a protein crystallizing gel having sodium chloride held in a gel-like material comprising a type of monomer selected from a group consisting of acrylamide, 2-acrylamide-2-methylpropanesulfonic acid, and methacryldimethylaminoethylmethyl chloride salt;
- a protein crystallizing gel having MPD held in a gel-like material containing dimethylacrylamide;
- a protein crystallizing agent having sodium/potassium phosphate held in a gel-like material containing 2-acrylamide-2-methylpropanesulfonic acid;
- a protein crystallizing gel having ammonium sulfate held in a gel-like material containing methacryldimethylaminoethylmethyl chloride salt;
- a protein crystallizing gel having sodium malonate held in a gel-like material containing acrylamide; and
- a protein crystallizing gel having polyethylene glycol 6000 held in a gel-like material containing polyoxyethylene monoacrylate.

### BRIEF DESCRIPTION OF THE DRAWINGS

FIG. 1 is a schematic diagram showing a usage state of a protein crystallizing device of the present invention.
FIG. 2 shows an example of a protein crystallizing microarray used in the present invention.
FIG. 3 is a schematic diagram showing an example of the protein crystallizing device of the present invention.
FIG. 4 is a partial cross-sectional view of a plate used for the present invention.
FIG. 5 is a plan view of the plate used in the present invention.
FIG. 6 is a plan view showing a usage state of the plate and a sample filling aid in the present invention.
FIG. 7 is a plan view of a supporting body used in the present invention.
FIG. 8 is a plan view showing the usage state of the protein crystallizing device of the present invention.

### BRIEF DESCRIPTION OF THE REFERENCE SYMBOLS

12 Hollow fiber; 16 Crystallizing agent holding part; 18 Protein crystallizing microarray; 20 Supporting body; 24 Plate; 32 Crystallizing section; 34 Recessed part

### BEST MODE FOR CARRYING OUT THE INVENTION

Hereunder is a description of embodiments of the present invention, with reference to the drawings.

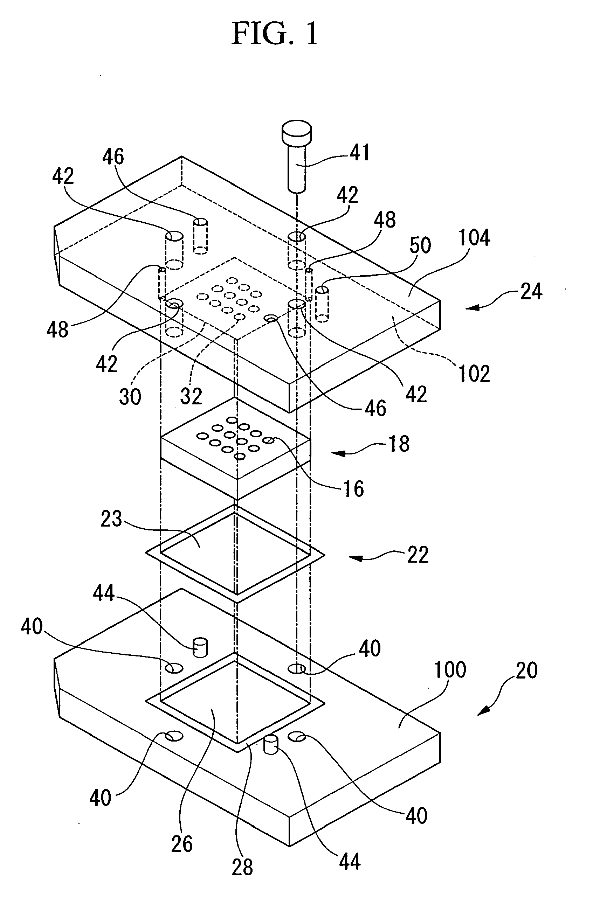

FIG. 1 is a schematic drawing showing an example of a protein crystallizing device of the present invention.

As shown in FIG. 1, the protein crystallizing device of this example comprises; a supporting body 20, a packing 22 made from a silicone rubber, a protein crystallizing microarray 18, and a plate 24.

The packing 22 has a hollow 23 of the same shape and area as those of the protein crystallizing microarray 18. The supporting body 20 is provided with a microarray supporting portion 26 of the same shape and size as those of the periphery of the packing 22. The microarray supporting portion 26 has a marking 28 of the same shape as that of the packing 22.

The packing 22 is set on the microarray supporting portion 26 so as to match the position of the marking 28. The protein crystallizing microarray 18 is stored and set in the hollow 23 of the packing 22 on the microarray supporting portion 26. Here, in order to accurately bring the crystallizing sections 32 provided on the plate 24, and the crystallizing agent holding parts 16 in the protein crystallizing microarray 18 into contact with each other, preferably the top face of the protein crystallizing microarray 18 and the top face of the supporting body 20 are at the same height.

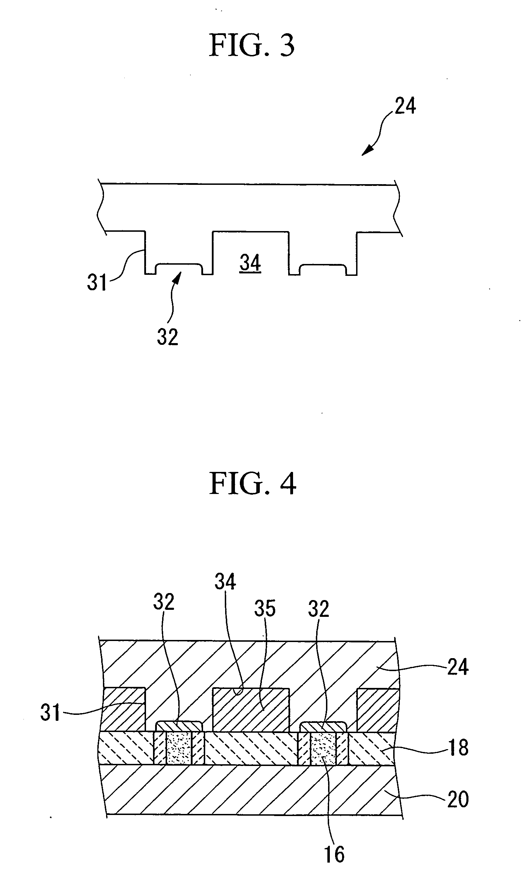

FIG. 3 is an enlarged cross-sectional view of the central part of the plate 24. FIG. 4 shows a partial cross-sectional view of the supporting body 20, the protein crystallizing microarray 18, and the plate 24 in the assembled state.

As shown in FIG. 3 and 4, the plate 24 has the crystallizing sections 32 in the arrangement corresponding to the respective crystallizing agent holding parts 16 in the protein crystallizing microarray 18, and recessed parts 34 provided between the crystallizing sections 32. The respective crystallizing sections 32 are respectively supported by the tips of the crystallizing section supporting parts 31 which are projecting parts with respect to the recessed parts 34 in the plate 24.

This plate 24 is laminated on the protein crystallizing microarray 18 supported by the supporting body 20. That is, as shown in FIG. 4, the crystallizing sections 32 are sealed by the crystallizing agent holding parts 16. Furthermore, the recessed parts 34 are respectively positioned between the crystallizing sections 32 supported by the crystallizing section supporting parts 31, and the crystallizing sections 32 similarly supported by the crystallizing section supporting parts 31.

Hereunder, the face of the supporting body 20 which supports the protein crystallizing microarray 18 is called a microarray supporting face 100. The face of the plate 24 which is in contact with the protein crystallizing microarray 18 is called a reaction face 102, and the opposite face thereof is called an outer face 104.

In the plate 24, the outer edge of the region where the crystallizing sections 32 are arranged becomes a recessed part. Consequently, the recessed region of a fixed area is formed in the plate 24. Hereunder, the recessed region on the reaction face of the plate 24 where the crystallizing sections 32 are arranged is called a seal portion 30.

In this example, the protein crystallizing microarray 18 is set on the microarray supporting portion 26 in the supporting body 20. However, the protein crystallizing microarray 18 may be set so that the crystallizing agent holding parts 16 can correspond to the crystallizing sections 32 so as to seal the crystallizing sections 32. That is, the protein crystallizing microarray 18 may be adhered onto the supporting body 20, or may be laminated on the bottom of a microarray supporting portion 26 without being adhered thereto by forming the microarray supporting portion in a recessed shape.

The material and the shape of the supporting body 20 are not specifically limited as long as it is capable of fixing the protein crystallizing microarray 18. As to the material of the supporting body 20, preferably an optically transparent material is used, since the generated crystal can be rapidly and simply confirmed as they are in the crystallizing device and the growth process of the crystal can be observed with time. Examples of the optically transparent material include an acrylic resin, a polycarbonate resin, a polystyrene resin, a polydimethylsiloxane (PDMS), and a glass.

The supporting body 20 has a marking for determining the fix position of the protein crystallizing microarray 18. The marking method is not specifically limited as long as it is capable of accurately fixing the protein crystallizing microarray 18. Examples thereof include a dent having a similar shape and area to those of the protein crystallizing microarray 18, and a dot painted in a position on the supporting body 20 corresponding to a predetermined position of the protein crystallizing microarray 18.

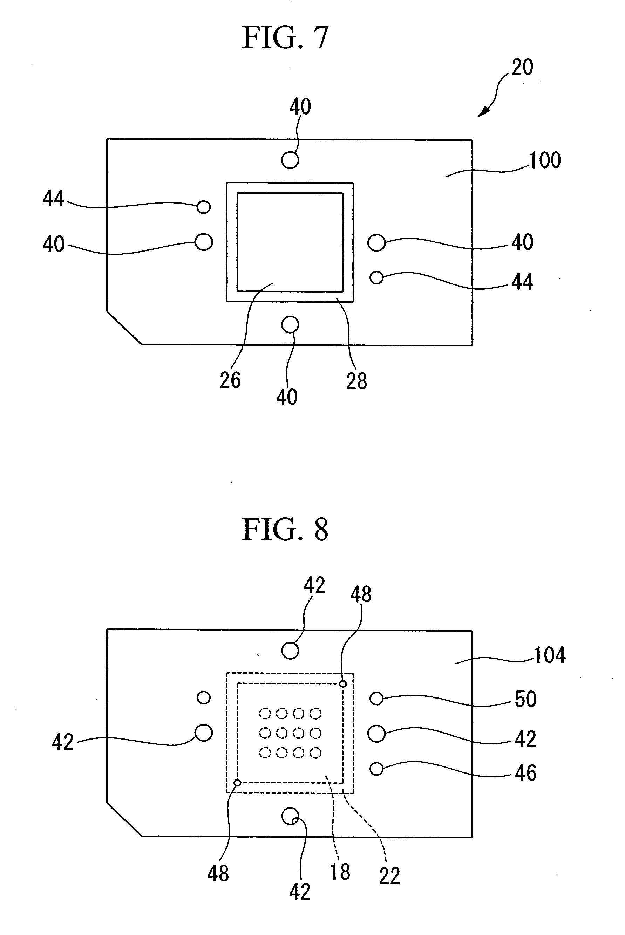

In this example, as shown in FIG. 7, the supporting body 20 is further provided with plate positioning holes 44 by which the crystallizing sections 32 correspond to the crystallizing agent holding parts 16 when the plate 24 and the supporting body 20 are laminated.

The protein crystallizing microarray 18 has at least two crystallizing agent holding parts 16 which hold a protein crystallizing agent.

As to the crystallizing agent holding part 16, a porous body (hereunder, called a holding phase) such as a gel capable of holding a protein crystallizing agent, having a protein crystallizing agent held therein, may be used. As shown in FIG. 4, the holding phase may be anything as long as the protein crystallizing agent is moved from the holding phase to the crystallizing sections 32 when the protein-containing samples filled in the crystallizing sections 32 are brought into contact with the crystallizing agent holding parts 16.

As to the holding phase, preferably a gel is used. By using a gel, the movement of the protein crystallizing agent from the crystallizing agent holding parts 16 to the protein-containing samples filled in the crystallizing sections 32 can be controlled at an appropriately slow speed. Therefore, a stable crystallization can be realized.

The material of the gel is not specifically limited, however an employable example thereof includes a gel having: one or more types of monomers such as acrylamide, N,N-dimethylacrylamide, N-isopropylacrylamide, N-acryloylaminoethoxyethanol, N-acryloylaminopropanol, N-methylolacrylamide, N-vinylpyrrolidone, hydroxyethyl methacrylate, (meth)acrylic acid, and allyl dextrin; and a polyfunctional monomer such as methylenebis(meth)acrylamide, and polyethylene glycol di(meth)acrylate, copolymerized for example, in an aqueous medium. In addition, as to the gel, employable examples include a gel such as agarose, alginic acid, dextran, polyvinyl alcohol, and polyethylene glycol, and a gel having them crosslinked.

In order to hold the gel as above in the protein crystallizing microarray 18, for example, a liquid containing a monomer such as acrylamide, a polyfunctional monomer, and an initiator serving as the constituents of the gel may be injected into the container so as to be polymerized and gelatinized. As to the method of gelatinization, in addition to the method of copolymerizing under the existence of the polyfunctional monomer, there may be a method of using a crosslinking agent after copolymerizing without the existence of the polyfunctional monomer. Moreover, if agarose is used as the gel material, the gelatinization may be performed by temperature reduction.

In the case of holding the protein crystallizing agent in a gel, the method is not specifically limited. For example, the protein crystallizing agent and the above polymeric monomer are previously mixed and introduced into a suitable container, then the polymerization reaction is performed to form a gel. As a result, the protein crystallizing agent can be held in the gel. Here, the protein crystallizing agent may be impregnated into porous particles and the like, and the particles may be included in the gel.

The material and the shape of the protein crystallizing microarray 18 are not specifically limited, as long as a large number of reacting substances are arranged in an alignment and the observation of the crystal precipitation is not interfered.

Examples of the material of the protein crystallizing microarray 18 include a glass, a resin, and a metal. Moreover, a complex formed by combining these materials may be used. However, in order to readily confirm the presence/absence of the precipitation of protein crystals, preferably a material having a high optical transparency is used.

Examples of the shape of the protein crystallizing microarray 18 include a circle, a square, and a rectangle. Moreover, the thickness thereof may be optionally selected considering the improvement of the efficiency of crystallization, and the facilitation and speeding-up of the observation of crystal precipitation. For example, it may be 0.1 to 5 mm, preferably 0.2 to 2 mm.

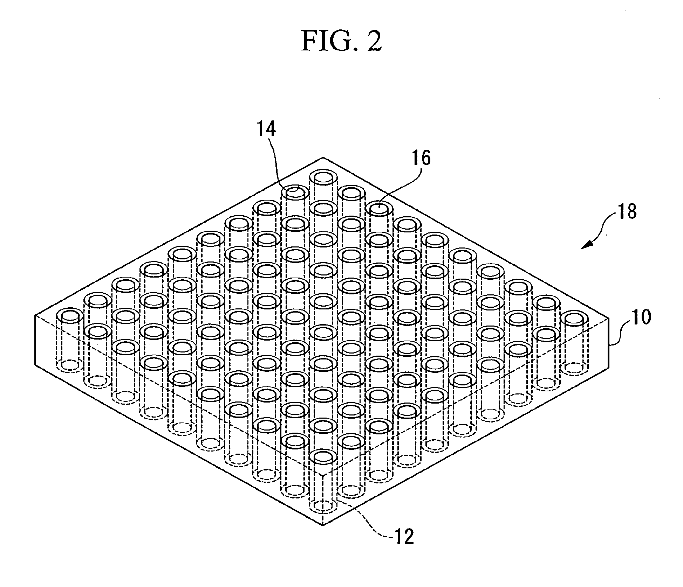

FIG. 2 shows a microarray in which a plurality of hollow tublar bodies are arranged, as a preferred example of the protein crystallizing microarray used for the protein crystallizing device of the present invention. As shown in FIG. 2, at least two hollow fibers 12 as the hollow tublar bodies are arranged in sections and fixed into the substrate 10. These hollow fibers 12 have hollows 14. These hollows 14 are filled with a gel holding the protein crystallizing agent, forming the crystallizing agent holding parts 16. That is, at least two crystallizing agent holding parts 16 are arranged in sections in the substrate 10, constituting the protein crystallizing microarray 18.

Examples of the material of the hollow fiber 12 include a homopolymer of a methacrylate monomer such as methyl methacrylate, ethyl methacrylate, and butyl methacrylate, and an acrylate monomer such as methyl acrylate, and ethyl acrylate, or a copolymer thereof, polystyrene, polyethylene, polypropylene, nobornene/ethylene copolymer, polyethylene terephthalate, polycarbonate, and a glass.

The internal surface of the hollow fiber 12 may be untreated to be used. Moreover, as required, it may be applied with a plasma treatment, a radiation treatment such as y rays and electron beams, and the like. Furthermore, the hollow fiber 12 may be introduced with reactive functional groups as required.

The outer diameter of the hollow fiber 12 is preferably 2 mm or less in order to increase the number of the crystallizing agent holding parts 16 per unit area. Less than 0.7 mm is more preferred. Moreover, the inner diameter of the hollow fiber 12 is appropriately selected within a range of the outer diameter.

As to the method of arranging at least two hollow fibers 12 in sections and fixing into the substrate 10, the following methods can be used. That is, firstly the hollow fibers 12 are arranged in parallel at predetermined intervals. These hollow fibers 12 are bundled then adhered, so as to form a fiber arrangement body (three dimensional arrangement body). Using a device for making sections such as a microtome, the obtained three dimensional arrangement body is cut along a direction crossing over the fiber axis, preferably a direction perpendicular to the fiber axis. As a result, a microarray comprising a thin piece (FIG. 2) having a cross section of the hollow fiber arrangement body can be obtained. The thickness of the thin piece may be normally 100 to 5000 µm to be used, and preferably 200 to 2000 µm.

At this time, by orderly arranging the hollow fibers 12 and adhering them by a resin bond or the like, for example, a hollow fiber arrangement body in which the hollow fibers 12 are orderly arranged in an alignment in the lengthwise and widthwise directions, can be obtained. The shape of the hollow fiber arrangement body is not specifically limited. Normally, it is formed into a square shape, rectangular shape, a circular shape, or the like by orderly arranging the fibers.

"Orderly" means to arrange in a good order so that the number of fibers contained in a frame of a fixed size becomes constant. For example, in the case where fibers having the diameter of 1 mm are bundled so as to be arranged into a square shape having the cross section of the length of 10 mm and the width of 10 mm, the number of the fibers contained in one side of the square frame (1 cm²) is set to 10. The 10 fibers are bundled into one row to make a sheet of one layer. Then, the sheet is laminated to make 10 layers. As a result, the total of 100 fibers, 10 in the lengthwise by 10 in the widthwise, can be arranged. However, the method of orderly arranging the fibers is not limited to the above method of laminating sheets.

As to the protein crystallizing microarray 18, by using the above microarray having a plurality of hollow tublar bodies in an array as described above, the thickness of the protein crystallizing microarray 18, the volume of the crystallizing agent holding part 16, the type and the concentration of the protein crystallizing agent held therein, and the like can be satisfactorily controlled, and the protein crystallizing device can be efficiently manufactured.

The protein crystallizing microarray 18 is preferably preserved by sealing by an airtight container, a seal, or the like which prevents the contact with the air. Examples of the material of the airtight container includes a polymeric material having a small transmissivity of gas or water, a glass, and a metal. Moreover, such a microarray is preferably preserved at a low temperature, particularly in the case of a long term preservation, it may be cryopreserved.

Furthermore, the respective crystallizing agent holding parts 16 are preferably made from gels prepared in different protein crystallization conditions. As a result, in the case where the screening of the crystallization conditions is performed, the screening can be rapidly performed in one microarray.

Here, the protein crystallization conditions mean the type and the concentration of the protein crystallizing agent, the holding phase for holding the protein crystallizing agent, for example, pH of the solidified gel in which the protein crystallizing agent is held, the composition of the gel, and the degree of cross-linkage thereof, the temperature of the crystallizing agent holding part 16 and the crystallizing section 32, the time, the cooling profile, and the like.

Examples of the type of the protein crystallizing agent include a precipitant, a pH buffer, and an optional combination thereof.

Examples of the precipitant include sodium chloride, polyethylene glycol, 2-methyl-2,4-pentanediol, and ammonium sulfate. Examples of the pH buffer include sodium acetate trihydrate, potassium phosphate, imidazole, sodium citrate, and sodium cacodylate. They may be solely used, or two types or more in optional combination may be used. Furthermore, as to the protein crystallizing agent, as a commercial product, Emerald BioStructures's "WIZARD II", Hampton Research's "Crystal screen", "Grid Screen", and the like may be used.

As to the concentration of the protein crystallizing agent, although it depends on the type of the protein crystallizing agent to be used, for example, in the case of a precipitant comprising polyethylene glycol, it is 5 to 50 volume %, preferably 10 to 35 volume %. On the other hand, in the case of the pH buffer, it is 0.05 to 0.5 mol/L, preferably 0.1 to 0. 2 mol/L.

As described above, by preparing the respective crystallizing agent holding parts 16 in respectively different protein crystallization conditions, the screening of the crystallization conditions for the target protein can be rapidly performed. Here, for example, it is preferable to set the concentrations of the protein crystallizing agent in many steps. Specifically, if a precipitant comprising sodium chloride is used, the dilution row is made such as in 5 steps, 10 step, and 20 steps within a range between 0.5 and 4.0 mol/L. Then the protein crystallizing agents of the respective concentrations can be filled in the crystallizing agent holding parts 16.

The protein crystallizing microarray 18 preferably has 10 to 1000 of crystallizing agent holding parts 16, in order to perform the crystallization in greater number of crystallization conditions at once. For example, in a normal screening of the protein crystallization conditions, it is necessary to examine about 800 of crystallization conditions. Consequently, the number of the crystallizing agent holding parts is preferably 10 or more. On the other hand, if the protein crystallizing microarray 18 has more than 1000 of the crystallizing agent holding parts 16, the interval between the crystallizing agent holding parts 16 becomes very narrow, being inferior in the handling efficiency.

In order to readily confirm the protein precipitation, preferably the protein crystallizing microarray 18 is made from a material having a high optical transparency.

In this example, the packing 22 is made from a silicone rubber. The material of the packing is not specifically limited as long as it is suitable for using in combination with other components in the protein crystallizing device (for example, the protein crystallizing microarray 18, the plate 24, and the supporting body 20).

The plate 24 has the crystallizing sections 32 corresponding to the crystallizing agent holding parts 16 and capable of being filled with the protein-containing samples, and the recessed parts 34 provided between these crystallizing sections 32. That is, the same number of the crystallizing sections 32 as that of the crystallizing agent holding parts 16 are provided onto the plate 24.

The plate 24 is preferably formed with for example, a sample filling aid positioning hole 50 as a positioning section which matches the position with a sample filling aid described later.

In this example, the supporting body 20 is further provided with plate positioning members 44 which make the crystallizing sections 32 and the crystallizing agent holding parts 16 correspond to each other when the plate 24 and the supporting body 20 are laminated. The plate 24 is formed with plate positioning holes 46 corresponding to the plate positioning members 44 provided on the supporting body 20.

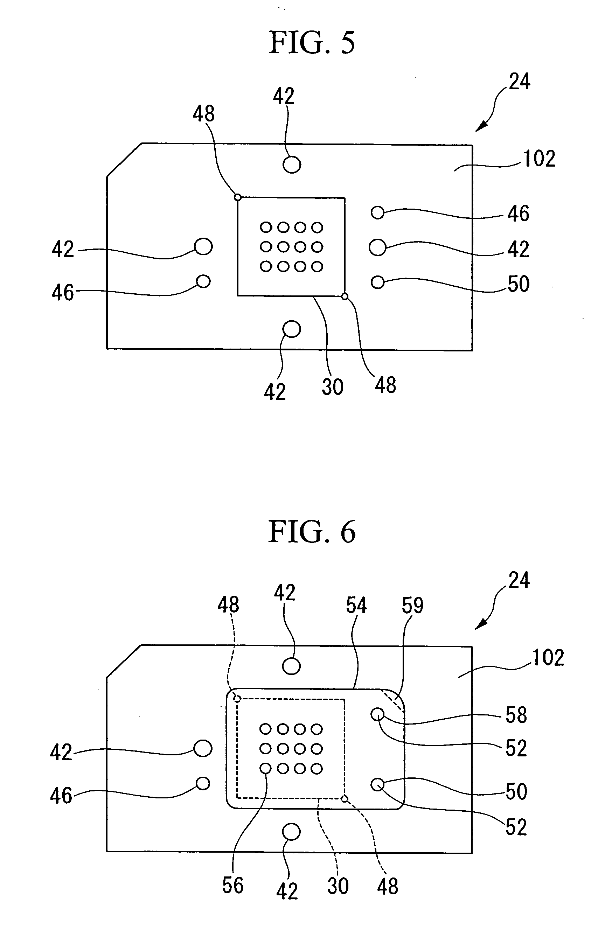

As shown in FIG. 1 and 5, in this example, furthermore, sealant inlets 48 piercing from the outer face 104 to the reaction face 102 of the plate 24 are bored. As shown in FIG. 5, in this example, two sealant inlets 48 are bored on the opposite sides to each other via the seal portion 30. As a result, if a sealant is injected from one of the sealant inlet 48 into the seal portion 30, since the air existing in the seal portion 30 at the beginning is exhausted from the other sealant inlet 48, the crystallizing sections 32 positioned between the recessed parts 34 in the seal portion 30 can be more reliably sealed.

The material and the shape of the plate 24 are not specifically limited as long as it can be overlaid on the protein crystallizing microarray 18, and optionally used considering the shape of the protein crystallizing microarray 18. Examples of the material of the plate include a glass, a resin, and a metal. The material can be optionally selected from them, which may be solely used or two types or more in combination may be used. In order to observe the protein crystallization state in the crystallizing sections 32, overlapped parts corresponding to the respective crystallizing agent holding parts 16 when the plate 24 is overlaid on the protein crystallizing microarray 18, are preferably optically transparent. Therefore, as to the material of the plate 24, an optically transparent material, for example, a transparent resin, a glass, and the like are preferably used.

Preferably, the plate 24 further has a crystal collection mechanism which collects precipitated crystals in the crystallizing sections 32. Using such a crystal collection mechanism, the precipitated crystals in crystallizing sections 32 are collected and used as seed crystals. Then, by performing the crystal growth process in a consecutive manner, crystals endurable against the X-ray structure analysis can be rapidly obtained. Of course, if the precipitated crystals are crystals endurable against the X-ray structure analysis as they are, they are collected and used for analysis.

An example of such a crystal collection mechanism includes a mechanism wherein the seal portion 30 is made from a member independent of the plate 24 and is connected to the plate 24 by a hinge mechanism, and the seal portion 30 can be opened to the outer face 104 side as required.

The shape of the crystallizing section 32 is not specifically limited as long as the protein-containing samples can be filled, and sealed when they come into contact with the crystallizing agent holding part 16.

If the object is to perform the screening of the crystallization conditions for the target protein, the capacity of the crystallizing section 32 is preferably less than 0.5 µl in order to reduce the protein amount required for the screening of the crystallization conditions. On the other hand, if the object is to use crystals precipitated here as seed crystals for forming crystals to be supplied to X-ray structure analysis, or to be supplied to structure analysis in a consecutive manner, the capacity of the crystallizing section 32 is preferably 0.5 µl or more. The reason is that in this case large crystals of 0.1mm or more capable of performing X-ray structure analysis, can be obtained. Regarding the relation between the area of the crystallizing agent holding part 16 and that of the crystallizing section 32, the condition may be such that the area of the face where the crystallizing section 32 comes into contact with the crystallizing agent holding part 16 allows the crystallizing agent held in the crystallizing agent holding part 16 to move to the crystallizing section 32 so as to be reacted with the protein-containing samples filled in the crystallizing section 32. The crystallizing section 32 may be larger than the crystallizing agent holding part 16. However, preferably the crystallizing sections 32 are not in contact with the substrate 12 constituting the protein crystallizing microarray 18.

In this example, furthermore as shown in FIG. 4, all of the recessed parts 34 provided between the crystallizing sections 32 are filled with the sealant 35.

The sealant 35 may be anything as long as it is not mutually dissolved with the protein-containing samples, and does not erode the members such as the plate 24, the packing 22, and the substrate 12 constituting the protein crystallizing microarray 18. For example, paraffin oil, silicone oil, and the like can be used.

Even if the sealant 35 is not used, by having the recessed parts 34, the protein-containing samples can be prevented from entering the adjacent sections. However, if the sealant 35 is used, the mixing of the protein-containing samples with each other and the entry thereof into the adjacent crystallizing sections 32 can be further reliably prevented, and the evaporation of the protein-containing sample can be prevented.

In this example, furthermore, there is shown a mechanism which presses the plate 24 and the protein crystallizing microarray 18 supported by the supporting body 20 into contact with each other. Specifically, as shown in FIG. 1, there are provided a first screw hole 40 piercing the supporting body 20 and a second screw hole 42 piercing the plate 24 and corresponding to the first screw hole 40. As shown in FIG. 1, a screw 41 is screwed into these first screw hole 40 and second screw hole 42. By using such a mechanism, the crystallizing sections 32 can be held in a sealed condition more stably.

In the protein crystallizing device of this example, there may be further provided a detection mechanism which monitors the protein crystallization in the crystallizing sections 32.

An example of the detection mechanism includes a detection mechanism comprising a microscope set on the outer face 104 of the plate 24 and a CCD camera installed in the microscope. In such a detection mechanism, the behavior of the crystal precipitation is captured and recorded by the CCD camera installed in the microscope, and the recorded image data is processed, by which the success and failure of the crystallization can be rapidly judged. Consequently, the protein crystallization conditions can be rapidly determined.

Here, an example of a suitable method of application of the protein crystallizing device of this example is shown.

### [Filling of protein-containing sample]

In this example, using the sample filling aid, the protein-containing sample can be filled in the crystallizing sections 32.

### (Crystallizing sections)

When carrying out the present invention, if the object is to crystallize a protein, the capacity of the crystallizing section 32 is preferably 0.5 µl or more. If the object is to perform screening of the protein crystallization conditions more rapidly, the capacity of the crystallizing section 32 is preferably less than 0.5 µl.

### (Protein-containing sample)

In the present invention, the protein-containing sample means a sample containing a protein (hereunder, called a target protein) which is to be crystallized, or of which the crystallization conditions are to be specified. Examples of the protein include a natural or synthetic peptide, polypeptide, protein, or protein complex. Preferably, after producing these substances from a natural or synthetic material by extraction/isolation, or genetic engineering methods or chemical synthesis methods, the target protein is purified using normal purification techniques such as solvent extraction, column chromatography, liquid chromatography, and the like in combination.

The concentration and purity of a protein in the protein-containing sample is also one factor of the protein crystallization conditions. Therefore, a series of the protein-containing samples having several stepwise concentrations and purities may be prepared and reacted with the protein crystallizing agent. For example, the protein concentration is preferably varied in several steps within a range between 1 to 50 mg/ml. Moreover, the amount of the protein-containing sample to be reacted with the protein crystallizing agent may be suitably varied according to the capacity and the number of the crystallizing sections 32 to be used, and the like. The viscosity of the protein-containing sample is not specifically limited as long as the protein-containing sample does not leak from the crystallizing sections 32 when the plate 24 is held by orienting the crystallizing sections 32 holding the protein-containing samples downward.

In addition to the target protein, the protein-containing sample may contain a protein solubilizer which aids to dissolve the protein, a stabilizer such as a reductant, and the like. An example of the protein solubilizer includes a surfactant which dissolves membrane proteins. If a surfactant is used, proteins with a low water solubility such as membrane proteins can be satisfactorily dispersed in the protein-containing sample. Consequently, the protein crystallization can be efficiently performed by applying the protein crystallizing device of this example.

Firstly, the plate 24 as shown in FIG. 1 is set still by orienting the reaction face 102 upward as shown in FIG. 5. The sample filling aid positioning hole 50 and the plate positioning holes 46 bored in the plate 24 are inserted with cylindrical guide pins 52 having the same diameters as those of the sample filling aid positioning hole 50 and the plate positioning holes 46 one by one, so as to fix them.

Next, as shown in FIG. 6, on this reaction face 102 is set a thin-planar sample filling aid 54 having punched holes 56 in an array corresponding to the crystallizing sections 32 on the plate 24, and two positioning holes 58 serving as a positioning mechanism for making the punched holes 56 correspond to the crystallizing sections 32 on the plate 24. At this time, the guide pins 52 that have been respectively fixed into the sample filling aid positioning hole 50 and the plate positioning hole 46 in the plate 24 are matched with the positioning holes 58 bored in the sample filling aid 54, and inserted thereinto. Then, the sample filling aid 54 is set on the plate 24. As a result, the sample filling aid 54 is set so that the punched holes 56 in the sample filling aid 54 correspond to the crystallizing sections 32 in the plate 24.

Then, a protein-containing sample of the amount that can be spread throughout the whole region arranged with the punched holes 56 in the sample filling aid 54 is put on the sample filling aid 54 that has been set on the plate 24.

Furthermore, by rubbing the surface of the sample filling aid 54 using a spatula and the like, the protein-containing sample is filled into the crystallizing sections 32 corresponding to the punched holes 56.

Then, the guide pins 52 are pushed out from the reaction face 102 side to the outer face 104 side of the plate 24, and the sample filling aid 54 is removed from the plate 24. As a result, the protein-containing sample can be filled in all of the crystallizing sections 32.

As described above, the sample filling aid 54 is set on the reaction face 102 of the plate 24, and the protein-containing sample is added thereon, by which the protein-containing sample can be accurately filled in the crystallizing sections 32.

The sample filling aid 54 is preferably made from a stainless steel from the aspects of the facileness of molding, the strength, the salt tolerance, and the like. Moreover, the sample filling aid 54 is preferably provided with a portion handled by tweezers and the like, so that the sample filling aid 54 can be readily taken our after the protein-containing sample is filled in the crystallizing sections 32. For example, a bent portion 59 is preferably provided by bending one end of the sample filling aid 54 upward.

Here, the guide pins 52 are respectively fixed into the sample filling aid positioning hole 50 and the plate positioning holes 46. However, in the plate 24, a plurality of sample filling aid positioning holes 50 may be formed independently from the plate positioning holes 46.

The method of filling the protein-containing sample into the crystallizing sections 32 may be anything as long as the protein-containing sample can be filled in all of the crystallizing sections 32 by the same amount, and can be kept from being adhered onto the components other than the crystallizing sections 32. For example, using a pipetter and the like rather than using the sample filling aid 54, the protein-containing sample can be filled in the respective crystallizing sections 32. Moreover, an automated equipment for sample addition may be also used.

However, in order to rapidly and economically fill the protein-containing sample, the method of using the sample filling aid 54 is preferred.

If a pipetter is used for filling the protein-containing sample, rather than using a mechanism which discharges the protein-containing sample that has been drawn and held in the pipetter, it is preferred to bring a minute amount of a droplet that has been adhered onto the tip of the pipetter into contact with the vicinity of the crystallizing section 32.

### [Assembling of protein crystallizing device]

As shown in FIG. 1, the packing 22 is set to match the marking 28 on the supporting body 20. Then, the protein crystallizing microarray 18 is set on the supporting body 20 so as to be stored in the hollow 23 of the packing 22.

The plate 24 having the protein-containing samples filled in the crystallizing sections 32 is held by orienting the reaction face 102 downward, and then laminated on the protein crystallizing microarray 18 supported by the supporting body 20.

At this time, the setting is such that the plate positioning members 44 provided on the supporting body 20 are inserted through the plate positioning holes 46 bored in the plate 24.

In this example, on the protein crystallizing microarray 18 supported by the supporting body 20, the plate 24 is laminated by orienting the reaction face 102 downward. However, it may be such that the plate 24 having the protein-containing sample filled in the crystallizing sections 32 is set still by orienting the reaction face 102 upward, and the supporting body 20 supporting the protein crystallizing microarray 18 is laminated on the plate 24 by orienting the microarray supporting face 100 downward. It is preferable if the plate 24 is set by orienting the outer face 104 upward, since the injection of the sealant from the sealant inlets 48, or the observation of crystals precipitated in the crystallizing sections 32 can be readily performed.

The plate 24 is held by pressing against the supporting body 20, and while it is held, the screw 41 is screwed into the first screw hole 40 and the second screw hole 42, so as to the press plate 24 and the protein crystallizing microarray 18 supported by the supporting body 20 into contact with each other.

Then, using a microscope, the state of filling of the protein-containing samples is confirmed.

After confirming that the protein-containing samples are filled in the respective crystallizing sections 32, as shown in FIG. 5 and 8, the sealant is filled from the sealant inlet 48 bored in the plate 24 into the seal portion 30 and the recessed parts 34 using a pipet and the like. As a result, the protein-containing samples leaked to positions in the protein crystallizing microarray 18 other than the crystallizing agent holding parts 16 can be replaced by the sealant. Then, the contamination and the condition change due to the movement of the protein-containing samples between the adjacent crystallizing sections 32 can be further reliably prevented. Furthermore, the protein-containing samples can be prevented from being evaporated. The injection amount of the sealant is preferably the maximum amount that can be spread throughout the recessed parts 34 in the whole region of the seal portion 30, but can not be leaked from one sealant inlet 48 when the sealant is injected from the other sealant inlet 48 as shown in FIG. 5 and 8.

### [Screening of protein crystallization conditions]

Using the assembled protein crystallizing device, the screening of protein crystallization conditions can be performed to obtain the optimum crystallization conditions for the target protein. Moreover, crystals suitable for the structure analysis can be produced using the precipitated crystals.

After reacting the protein crystallizing agent and the protein-containing samples in the protein crystallizing device, the protein crystallizing device is set still over a sufficient time allowing the protein to be precipitated under a certain temperature condition in a sealed condition or in the air.

The "sufficient time allowing the protein to be precipitated" is about 1 hour to 10 days although it differs according to specific proteins, concentrations, and crystallization conditions. If no crystal is precipitated after 30 days or more, it is assumed that the crystallization conditions are not appropriate. Moreover, since the temperature condition is one factor of the protein crystallization conditions, the crystallization may be performed by varying the temperature condition. The temperature condition is preferably set in a plurality of steps such as 4°C, 15°C, 18°C, and 22°C.

After the passage of sufficient time allowing the protein to be precipitated, the protein crystal precipitation is observed. At this time, it is preferably observed from the outer face 104 side of the plate 24 by for example, an optical microscope.

In this manner, the optimum crystallization conditions for the target protein can be determined.

### [Production of crystal for structure analysis]

If the capacity of the crystallizing section 32 is set 0.5 µl or more with the object of protein crystallization, in the crystallizing sections 32 having precipitated crystals among all of the crystallizing sections 32, the crystals therein are further allowed to be grown, or the precipitated crystals are collected from the crystallizing sections 32 to be used as seed crystals, by which the crystals for structure analysis can be produced by a publicly known protein crystallizing method in the crystallization conditions similar to those of the crystallizing sections 32 from which the seed crystals were collected. Examples of the publicly known protein crystallizing method include a hanging drop method, a sitting drop method, a dialysis, and a batch method.

If the capacity of the crystallizing section 32 is set less than 0.5 µl with the object of more rapid screening of the protein crystallization conditions, crystals for structure analysis can be obtained by performing the crystallization of the target protein in the obtained optimum crystallization conditions. At this time, as to the method of obtaining crystals for structure analysis, a publicly known method such as a vapor diffusion method and a hanging drop method can be used.

By collecting the obtained crystals for structure analysis, and supplying the collected crystals to the X-ray structure analysis by a publicly known method, the steric structure of the target protein can be determined.

In the protein crystallizing device of this example, after the protein-containing samples are filled in the crystallizing sections 32, the plate 24 and the protein crystallizing microarray 18 are laminated, by which the crystallizing agent holding parts 16 and the crystallizing sections 32 filled with the protein-containing samples are overlaid to correspond to each other. As a result, the crystallizing agents held in the crystallizing agent holding parts 16 are moved into the crystallizing sections 32 and reacted with the protein. If the respective crystallizing agent holding parts 16 are set in different crystallization conditions, crystals are precipitated in the crystallizing sections 32 which are set in the suitable crystallization conditions for crystallizing the target protein.

As described above, in the protein crystallizing device of this example, the protein-containing samples are held in the crystallizing sections 32 and sealed by the crystallizing agent holding parts 16. Moreover, the respective crystallizing sections 32 are separated by the recessed parts 34. Consequently, the movement of the protein-containing sample between the crystallizing sections 32 and/or the mixing of the protein crystallizing agent between the crystallizing agent holding parts 16 can be prevented. Furthermore, the solution can be kept from being evaporated from the protein-containing samples and a minute amount of the protein-containing sample of "nl" level can be also stably held. Consequently, the protein crystallization can be performed with a high reliability. Moreover, the suitability of the crystallization conditions can be determined with an excellent reliability.

Furthermore, if the sealant is filled in the recessed parts 34, the mixing of the protein-containing samples with each other and the entry thereof into the adjacent crystallizing sections 32 can be further efficiently prevented. Moreover, the evaporation of the protein-containing sample can be prevented. Consequently, even if there is only a minute amount of the protein-containing samples, the crystallization conditions can be satisfactorily controlled.

Moreover, if the plate 24 and the supporting body 20 supporting the protein crystallizing microarray 18 are pressed into contact with each other, the crystallizing sections 32 can be stably sealed. Therefore, the crystallization can be performed with a higher reliability.

By appropriately selecting the capacity of the crystallizing section 32, both of the screening of the protein crystallization conditions and the production of the crystals for structure analysis can be rapidly and accurately performed.

Furthermore, if the sample filling aid is used, the screening of the protein crystallization conditions can be simply and rapidly performed.

The protein crystallizing device of the present invention can be suitably used for both of the screening of the protein crystallization conditions and the production of the protein crystal for structure analysis in the research and development, for example in the medical field, and the like.

The protein crystallizing gel of the present invention is characterized in that the protein crystallizing agent is evenly dispersed and dissolved in a gel by gelatinizing a solution containing the protein crystallizing agent and an unsaturated monomer.

The protein crystallizing agent is not specifically limited as long as it can reduce the solubility of the protein in the protein solution. Examples thereof as salts include ammonium sulfate, sodium chloride, sodium phosphate, potassium phosphate, lithium chloride, sodium malonate, sodium citrate, magnesium sulphate, lithium sulfate, sodium nitrate, cadmium sulfate, and sodium sulphate. Examples thereof as organic solvents include 2-methyl-2,4-pentanediol (hereunder, MPD), ethanol, isopropanol, dioxane, methanol, tert-butanol, and n-propanol. Examples thereof as water soluble high molecular compounds include polyethylene glycol (hereunder, PEG), polyethylene glycol monoalkylether, and polyethyleneimine.

These precipitants may be solely used, or two types or more in combination may be used. Among them, particularly ammonium sulfate, sodium chloride, potassium/sodium phosphate, lithium chloride, sodium malonate, MPD, and PEG are suitable.

As a commercial product, Emerald BioStructures's "WIZARD II", Hampton Research's "Crystal screen", "Grid Screen", and the like may be used. The concentration of the precipitant for usage is preferably 0.1 to 5.0 mol/L for salts, 1 to 80 volume % for organic solvents, and 1 to 50% by weight for water soluble high molecular compounds.

The protein crystallization is preferably performed in a specific pH region, and a buffer may be used for maintaining the pH. The buffer to be used is not specifically limited. However, examples thereof include those containing citric acid, 2-(N-morpholino)ethanesulfonic acid, N-2-hydroxyethylpiperazine-N-ethanesulfonic acid, tris (hydroxymethyl) aminomethane, and N,N-bis(hydroxyethyl)glycine. They are solely or the mixture of two types of more are neutralized by an acid or an alkali and the like so as to adjust the predetermined pH as necessary. The pH is preferably within a range between 3.0 and 10.0, and more preferably a range between 4.0 and 9.0.

In the present invention, an unsaturated monomer is used as a gelatinizer. The unsaturated monomer is not specifically limited as long as a gel can be formed by polymerizing in an aqueous medium. However, it is preferably a (meth)acrylamide monomer or a (meth)acrylic monomer.

Preferably employable examples of the (meth)acrylamide monomer include (meth)acrylamide, N,N-dimethylacrylamide, N,N-diethylacrylamide, and other N,N-dialkylamino(meth)acrylamide, (meth)acrylamide methanesulfonic acid, (meth)acrylamide ethanesulfonic acid, 2-(meth)acrylamide 2-methylpropanesulfonic acid, and other (meth)acrylamide alkylsulfonic acid, dimethylaminopropyl(meth)acrylamide, diethylaminopropyl(meth)acrylamide, dimethylaminoethyl(meth)acrylamide, and other dialkylaminoalkyl(meth)acrylamide, dimethylaminopropyl(meth)acrylamide methyl chloride salt, diethylaminopropyl(meth)acrylamide methylethyl chloride salt, dimethylaminoethyl(meth)acrylamide methyl chloride salt, and other dialkylamino(meth)acrylamide quaternary ammonium salt.

Moreover, preferably employable examples of the (meth)acrylic monomer include 2-hydroxyethyl(meth)acrylate, 4-hydroxybutyl(meth)acrylate, 6-hydroxyhexyl(meth)acrylate, diethylene glycol mono(meth)acrylate, trimethylene glycol mono (meth) acrylate, polyethylene glycol mono (meth)acrylate, and other (meth)acrylate containing ahydroxyl group, dimethylaminoethyl(meth)acrylate, diethylaminoethyl(meth)acrylate, and other dialkylaminoalkyl(meth)acrylate, dimethylaminoethyl(meth)acrylate methyl chloride salt, diethylaminoethyl(meth)acrylate ethyl chloride salt, and other quaternary ammonium salt of dialkylaminoalkyl(meth)acrylate.

The monomer concentration is preferably 0.1 to 50% by mass, and more preferably 1 to 10% by mass with respect to the protein crystallizing agent solution of 100% by mass.

Moreover, the crosslinking monomer capable of copolymerising with the above monomer used as required in the present invention, is not specifically limited as long as it is a monomer having a polyfunctional radical polymerizing group. However, examples thereof include N,N'-methylenebis(meth)acrylamide, ethylene glycol di(meth)acrylate, diethylene glycol di(meth)acrylate, triethylene glycol di(meth)acrylate, polyethylene glycol di(meth)acrylate, trimethylolpropane tri(meth)acrylate, and trimethylolpropane ethylene oxide modified tri(meth)acrylate. Particularly preferred are N,N'-methylenebis(meth)acrylamide, ethylene glycol di(meth)acrylate, and polyethylene glycol di(meth)acrylate. The dosage of the crosslinking monomer is 0.01 to 10 mass parts with respect to the monomer of (A) group or (B) group. It is preferably 0.1 to 5 mass parts.

Regarding the protein crystallizing agent of the present invention, a transparent gel can be obtained by combining a specific precipitant and a specific unsaturated monomer. If the gel is transparent, the observation of the generated protein crystals is exceedingly facilitated. Therefore, the automization by the optical detection system is also facilitated.

The combination of the unsaturated monomer and the protein crystallizing agent capable of obtaining the transparent gel include:
(1) at least one type of monomer selected from a group consisting of acrylamide, 2-acrylamide-2-methylpropanesulfonic acid, and methacryldimethylaminoethylmethyl chloride salt, and sodium chloride;
(2) dimethylacrylamide and MPD;
(3) 2-acrylamide-2-methylpropanesulfonic acid and sodium/potassium phosphate,
(4) methacryldimethylaminoethylmethyl chloride salt and ammonium sulfate;
(5) acrylamide and sodium malonate, and
(6) polyoxyethylene monoacrylate and PEG6k.

The protein crystallizing agent of the present invention is prepared by heat polymerization of a solution containing at least a precipitant, a buffer, and an unsaturated monomer. Alternatively, it may be prepared by such that the gelatinization is performed by polymerizing under the existence of heat- and/or photo-radical polymerization initiator and the precipitant is evenly held in the gel. The polymerization is suitably performed under the existence of a radical polymerization initiator. Examples of the radical polymerization initiator suitably used in the solution include tert-butylhydroperoxide, hydrogen peroxide, ammonium persulfate, potassium persulfate, and other peroxide, 2,2'-azobis (2-amidinopropane) dihydrochloride, 2,2'-azobis (2-amidinobutane) dihydrochloride, 2,2'-azobis [2-(2-imidazoline-2-yl) propane] dihydrochloride, and other azo polymerization initiator. These radical polymerization initiators may be solely used, or the mixture of two types of more may be used. Moreover, a redox polymerization initiator which is the combination of the above peroxides and a reductant such as tertiary amines, sulfite salts, and ferrous iron salts, and furthermore a compatible polymerization initiator which is the combination of the redox polymerization initiator and the azo polymerization initiator may be also used.

Moreover, the polymerization may be also performed using a photo-radical polymerization initiator under a light source which gives light of a specific wavelength. In that case, the photo-radical polymerization initiator to be used is not specifically limited as long as it is discomposed by irradiation of light within a range of specific wavelengths, and generates radicals. Suitably employable examples include acylphosphine oxido, benzoin, benzoinalkylether, benzil, benzophenone, anthraquinone, and other initiator that is normally used for photopolymerization, in addition, 2,2'-azobis (2-methylpropionamidine) hydrochloride, sodium 4,4'-azobis (4-cyanovalerate), 2,2'-azobis[2-methyl-N-(2-hydroxyethyl) propionamide], and other azo polymerization initiator. Among them, one type or more may be optionally selected and used according to the light wavelength to be used. Moreover, regarding the specific wavelength, light within a range between 200 and 650 nm of wavelength is desirably used, considering two points of the photoabsorption by the monomer itself contained in the reaction liquid, and the light quantum energy used for the radical generation. Typical examples of the light source employable for the irradiation of light within a range between 200 and 650 nm of wavelength, include a high-pressure mercury-vapor lamp, a low-pressure mercury-vapor lamp, a metal halide lamp, a fluorescent chemical lamp, and a fluorescent blue lamp.

### EXAMPLES

### (Production of hollow fiber arrangement body)

As a component which supports the crystallizing agent holding parts, a hollow fiber arrangement body was produced.

Two perforated plates having a thickness of 0.1 mm in which the total of 100 holes having the diameter of 0.32 mm and the pitch of 0.42 mm were arranged by 10 in the lengthwise by 10 in the widthwise, were overlaid. 100 hollow fibers made from polyethylene (Mitsubishi Rayon's, the outer diameter of about 500 µm, the inner diameter of about 300 µm, and the length of about 100 cm) were inserted into the respective holes of these perforated plates. Next, in the condition where the hollow fibers were tensioned, two points of 10 cm and 40 cm from one end of the hollow fiber were fixed, to set the space between the two perforated plates 30 cm.

Next, three directions of the space between the two perforated plates were enclosed by plate like materials made from a silicone. From the open one direction, as a resin material, a mixture of a resin made from a polyurethane resin bond and a carbon black of 2.5% by mass with respect to the gross mass of the resin material was poured into the space between the two perforated plates. Then, it was set still at room temperature for a week to cure the resin. Then, the plate like materials that had been set between the perforated plates were removed, and the hollow fiber arrangement body was obtained.

### (Introduction and fixation of polymer gel into hollow fiber arrangement body)

A mixed solution composed of the following composition was prepared.
acrylamide : 3.7 mass parts
methylenebisacrylamide : 0.3 mass parts

### 2,2'-azobis (2-amidinopropane) dihydrochloride : 0.1 mass parts

The mixed solution and the hollow fiber arrangement body obtained by the above manner were put into a desiccator. In the condition where the longer ends of the free ends of the respective hollow fibers were soaked in this mixed solution, the internal pressure of the desiccator was reduced, so as to introduce the mixed solution into the hollows in the hollow fibers. Next, this hollow fiber arrangement body was moved into a sealed glass container inside of which was saturated with water vapour, where the polymerization reaction was performed at 80°C for 4 hours. As a result, the hollow fiber arrangement body having the acrylamide gels fixed in the hollows in the hollow fibers, was obtained.

### (Production of gel-filled hollow fiber arrangement body thin piece)

The acrylamide gel containing hollow fiber arrangement body obtained by the above manner was cut out into a thickness of about 2 mm along the direction orthogonal to the longitudinal direction of the hollow fiber using a microtome, so as to obtain the arrangement body thin piece in which the total of 100 hollow fibers having the hollows filled with a gel were orderly and squarely arranged by 10 in the lengthwise by 10 in the widthwise. FIG. 2 shows the gel-containing hollow fiber arrangement body thin piece obtained by the above process. As shown in FIG. 2, the hollows 14 in the hollow fibers 12 are filled with the acrylamide gel produced by the above manner.

### (Production of protein crystallizing microarray)

In the gel-containing hollow fiber arrangement body thin piece produced by the above manner, the acrylamide gels filled and held in the respective hollows 14 in the hollow fibers 12 were added with 1 µl of the protein crystallizing agents composed of A, B, C, D, E, F, G, H, I, and J solutions described below. By so doing, the protein crystallizing agents were held in the acrylamide gels to produce the protein crystallizing microarray 18.
A : 2.0 mol/L sodium chloride aqueous solution
B : 0.5 mol/L sodium chloride aqueous solution
C : 10 volume % polyethylene glycol (molecular weight 400) aqueous solution
D : 20 volume % polyethylene glycol (molecular weight 400) aqueous solution
E : 10 volume % polyethylene glycol (molecular weight 6000) aqueous solution
F : 20 volume % polyethylene glycol (molecular weight 6000) aqueous solution
G : 20 volume % 2-methyl-2,4-pentanediol aqueous solution
H : 20 volume % 2-methyl-2,4-pentanediol aqueous solution.
I : 0.5mol/L ammonium sulfate aqueous solution
J : 1.5mol/L ammonium sulfate aqueous solution

Hereunder, the crystallizing agent holding parts 16 holding the protein crystallizing agents of A to J are respectively called spots A to J.

### Screening of protein crystallization conditions

Using the protein crystallizing device shown in FIG. 1, screening of the crystallization conditions of lysozyme (Sigma-Aldrich's) was performed. As to the protein crystallizing microarray, the protein crystallizing microarray 18 produced by the above manner was used. As to the material of the supporting body 20 and the plate 24, acrylic resins were used.

### (Addition of protein-containing sample)

Using the sample filling aid 54 shown in FIG. 6, the crystallizing sections 32 were filled with the protein-containing samples. The sample filling aid 54 having the punched holes 56 in the arrangement corresponding to the crystallizing agent holding parts 16 in the protein crystallizing microarray 18 obtained above, was used.

The crystallizing section 32 is in a cylindrical shape with the diameter of 0.7 mm and the height of 0.15 mm having one end closed, and the capacity of the crystallizing section 32 was 105 nl.

As to the protein-containing sample, lysozyme-containing solution (80 mg/ml) was used.

Firstly, as shown in FIG. 6, the sample filling aid 54 was set on the plate 24, onto which 1.5 to 2 µl of the protein-containing sample was added so as to be spread throughout all of the punched holes 56 by a 20 µl autopipette. Next, by pushing the protein-containing sample onto the plate 24 from the top of the sample filling aid 54 by a spatula, the protein-containing samples were filled in all of the crystallizing sections 32 in the plate 24.

### (Assembling of protein crystallizing device, and screening of protein crystallization conditions)

Next, the plate 24 having the protein-containing samples filled in the crystallizing sections 32 was held by orienting the reaction face 102 downward, and laminated on the protein crystallizing microarray 18 supported by the supporting body 20. After confirming that the crystallizing sections 32 were filled with the protein-containing samples, paraffin oil (hereunder, called oil) as a sealant was injected into one sealant inlet 48 bored in the plate 24 from the outer face 104 side of the plate 24, using a pippet. The injection amount of the oil was such that the oil was spread throughout the whole region of the seal portion 30, but just before overflowing from the other sealant inlet 48.

Then, the protein crystallizing device was set still at 20°C for 3 hours. In a consecutive manner, the inside of the crystallizing sections 32 was observed by an optical microscope, resulting in that no lysozyme crystal was recognized in the spot B holding polyethylene glycol solution as a protein crystallizing agent, and that the most optimum columnar crystal for X-ray structure analysis was recognized in the spot A holding 2.0 mol/L sodium chloride solution.

### (Production of crystal for structure analysis)

Furthermore, based on the above result, using the 2.0 mol/L sodium chloride solution as a protein crystallizing agent, a lysozyme crystal was produced from the lysozyme-containing sample solution by a hanging drop method. At this time, the size of the obtained lysozyme crystal was 0.3 x 0.3 x 0.5 mm.

From the above result, the type and the concentration of the protein crystallizing agent were examined among the crystallization conditions of lysozyme, which revealed that 2.0 mol/L sodium chloride solution was the optimum crystallization condition. Using the revealed optimum crystallization condition, a crystal suitable for X-ray structure analysis could be obtained.

That is, the screening of the protein crystallization conditions was able to be performed rapidly and economically with a high reliability with a minute amount of protein-containing sample.

Regarding the buffer for the protein crystallizing gel of the present invention, the followings were used and prepared according to a usual method.
0.1M-citric acid buffer (pH 4.0)
0.1M-citric acid buffer (pH 5.0)
0.1M-MES (pH 6.0)
0.1M-HEPES (pH 7.0)
0.1M-Tris (pH 8.0)
0.1M-Bicine (pH 9.0)

### <Example 1>

0.48g of sodium chloride serving as a precipitant was weighed in a 10 ml measuring flask, and the volume was adjusted with 0.1M-citric acid buffer (pH 4:0) so as to prepare 1.2M-NaCl solution (it was used as A solution). Moreover, into 90g of 50% acrylamide solution (Mitsubishi Rayon's), was added and dissolved 5g of N,N'methylenebisacrylamide (hereunder, abbreviated MBAAm) and 5g of deionized water, so as to prepare 50% monomer solution (it was used as B solution). Furthermore, 10% solution (it was used as C solution) of 2,2'-azobis[2-(2-imidazoline-2-yl)propane] dihydrochloride (Wako Pure Chemical Industries' VA-044) serving as the water soluble polymerization initiator was prepared. 840 µl of A solution and 160 µl of B solution were injected and mixed in a 2 ml sample tube, so as to prepare a protein crystallizing agent solution (1.0M-NaCl, pH 4.0, monomer concentration 8%). 10 µl of C solution was further added and mixed well therein, then polymerized in a warm bath at 55°C for 3 hours, to obtain a transparent hydrogel.

The sample tube in which the hydrogel was formed in the above procedure, was added with 1 ml of 40 mg/ml lysozyme solution then incubated at 20°C for 24 hours, which resulted in the precipitation of lysozyme crystals in the lysozyme solution. The generation of the crystals was confirmed by naked eyes and a stereo microscope.

### <Example 2>

By the same procedure as that of Example 1 except that the concentration of A solution was changed to 2.4M, the crystallizing agent solution (2.0M-NaCl, pH 4.0, monomer concentration 8%) was prepared. Furthermore, by the same procedure, a transparent hydrogel was obtained. Moreover, the generation of crystals was confirmed by the same method. The result is shown in Table-1.

### <Example 3>

By the same procedure as that of Example 1 except that the concentration of A solution was changed to 3.6M, the crystallizing agent solution (3.0M-NaCl, pH 4.0, monomer concentration 8%) was prepared. Furthermore, by the same procedure, a transparent hydrogel was obtained. Moreover, the generation of crystals was confirmed by the same method. The result is shown in Table-1.

### <Example 4>

By the same procedure as that of Example 1 except that the concentration of A solution was changed to 4.8M, the crystallizing agent solution (4.0M-NaCl, pH 4.0, monomer concentration 8%) was prepared. Furthermore, by the same procedure, a transparent hydrogel was obtained. Moreover, the generation of crystals was confirmed by the same method. The result is shown in Table-1.

### <Examples 5 to 8>

By the same procedure as that of Examples 1 to 4 except that the buffer was changed to 0.1M-citric acid buffer (pH 5.0), the crystallizing agent solution was prepared. Furthermore, by the same procedure, a transparent hydrogel was obtained. Moreover, the generation of crystals was confirmed by the same method. The result is shown in Table-1.

### <Examples 9 to 12>

By the same procedure as that of Examples 1 to 4 except that the buffer was changed to 0.1M-MES buffer (pH 6.0), the crystallizing agent solution was prepared. Furthermore, by the same procedure, a transparent hydrogel was obtained. Moreover, the generation of crystals was confirmed by the same method. The result is shown in Table-1.

### <Examples 13 to 16>

By the same procedure as that of Examples 1 to 4 except that the buffer was changed to 0.1M-HEPES buffer (pH 7.0), the crystallizing agent solution was prepared. Furthermore, by the same procedure, a transparent hydrogel was obtained. Moreover, the generation of crystals was confirmed by the same method. The result is shown in Table-1.

### <Examples 17 to 20>

By the same procedure as that of Examples 1 to 4 except that the buffer was changed to 0.1M-Tris buffer (pH 8.0), the crystallizing agent solution was prepared. Furthermore, by the same procedure, a transparent hydrogel was obtained. Moreover, the generation of crystals was confirmed by the same method. The result is shown in Table-1.

### <Examples 21 to 24>

By the same procedure as that of Examples 1 to 4 except that the buffer was changed to 0.1M-Bicine buffer (pH 9.0), the crystallizing agent solution was prepared. Furthermore, by the same procedure, a transparent hydrogel was obtained. Moreover, the generation of crystals was confirmed by the same method. The result is shown in Table-1.

### <Example 25>

0.55g of polyethylene glycol (Wako Pure Chemical Industries' PEG6000, weight-average molecular weight : 6000) serving as a precipitant was weighed in a 10 ml measuring flask, and the volume was adjusted with 0.1M-citric acid buffer (pH 4.0) so as to prepare 5.5%-PEG solution (it was used as D solution). Moreover, into 95g of polyethylene glycol monoacrylate (NOF Corporation's BLEMMER AE90), was dissolved polyethylene glycol diacrylate (Shin-nakamura Chemical Corporation's NKESTERA-200), to prepare a monomer solution (it was used as E solution). Furthermore, 10% solution (it was used as C solution) of 2,2'-azobis[2-(2-imidazoline-2-yl)propane] dihydrochloride (Wako Pure Chemical Industries' VA-044) serving as the water soluble polymerization initiator was prepared. 920 µl of A solution and 80 µl of B solution were injected and mixed in a 2 ml sample tube, so as to prepare the protein crystallizing agent solution (5%-PEG, pH 4.0, monomer concentration 8%). 10 µl of C solution was further added and mixed well therein, then polymerized in a warm bath at 55°C for 3 hours, to obtain a transparent hydrogel. Hereunder, the generation of crystals was confirmed by the same method as that of Example 1. The result is shown in Table-1.

### <Example 26>

By the same procedure as that of Example 25 except that the concentration of D solution was changed to 11%, the crystallizing agent solution (10%-PEG, pH 4.0, monomer concentration 8%) was prepared. Furthermore, by the same procedure, a transparent hydrogel was obtained. Moreover, the generation of crystals was confirmed by the same method. The result is shown in Table-1.

### <Example 27>

By the same procedure as that of Example 25 except that the concentration of D solution was changed to 22%, the crystallizing agent solution (20%-PEG, pH 4.0, monomer concentration 8%) was prepared. Furthermore, by the same procedure, a transparent hydrogel was obtained. Moreover, the generation of crystals was confirmed by the same method. The result is shown in Table-1.

### <Example 28>

By the same procedure as that of Example 25 except that the concentration of D solution was changed to 33%, the crystallizing agent solution (30%-PEG, pH 4.0, monomer concentration 8%) was prepared. Furthermore, by the same procedure, a transparent hydrogel was obtained. Moreover, the generation of crystals was confirmed by the same method. The result is shown in Table-1.

### <Examples 29 to 32>

By the same procedure as that of Examples 25 to 28 except that the buffer was changed to 0.1M-citric acid buffer (pH 5.0), the crystallizing agent solution was prepared. Furthermore, by the same procedure, a transparent hydrogel was obtained. Moreover, the generation of crystals was confirmed by the same method. The result is shown in Table-1.

### <Examples 33 to 36>

By the same procedure as that of Examples 25 to 28 except that the buffer was changed to 0.1M-MES buffer (pH 6.0), the crystallizing agent solution was prepared. Furthermore, by the same procedure, a transparent hydrogel was obtained. Moreover, the generation of crystals was confirmed by the same method. The result is shown in Table-1.

### <Examples 37 to 40>

By the same procedure as that of Examples 25 to 28 except that the buffer was changed to 0.1M-HEPES buffer (pH 7.0), the crystallizing agent solution was prepared. Furthermore, by the same procedure, a transparent hydrogel was obtained. Moreover, the generation of crystals was confirmed by the same method. The result is shown in Table-1.

### <Examples 41 to 44>

By the same procedure as that of Examples 25 to 28 except that the buffer was changed to 0.1M-Tris buffer (pH 8.0), the crystallizing agent solution was prepared. Furthermore, by the same procedure, a transparent hydrogel was obtained. Moreover, the generation of crystals was confirmed by the same method. The result is shown in Table-1.

### <Examples 45 to 48>

By the same procedure as that of Examples 25 to 28 except that the buffer was changed to 0.1M-Bicine buffer (pH 9.0), the crystallizing agent solution was prepared. Furthermore, by the same procedure, a transparent hydrogel was obtained. Moreover, the generation of crystals was confirmed by the same method. The result is shown in Table-1.

**[Table 1]**

| | Precipitant | Monomer | pH | Gel property | Protein crystallization state |
|---|---|---|---|---|---|
| Ex. 1 | 1.0M-NaCl | Acrylamide | 4.0 | transparent gel | crystal |
| Ex. 2 | 2.OM-NaCl | Acrylamide | 4.0 | transparent gel | crystal, precipitation mixture |
| Ex. 3 | 3.0M-NaCl | Acrylamide | 4.0 | transparent gel | crystal, precipitation mixture |
| Ex. 4 | 4.0M-NaCl | Acrylamide | 4.0 | transparent gel | crystal, precipitation mixture |
| Ex. 5 | 1.0M-Nacl | Acrylamide | 5.0 | transparent gel | crystal |
| Ex. 6 | 2.0M-NaCl | Acrylamide | 5.0 | transparent gel | crystal |
| Ex. 7 | 3.0M-NaCl | Acrylamide | 5.0 | transparent gel | crystal, precipitation mixture |
| Ex. 8 | 4.0M-NaCl | Acrylamide | 5.0 | transparent gel | precipitation |
| Ex. 9 | 1.0M-NaCl | Acrylamide | 6.0 | transparent gel | crystal |
| Ex. 10 | 2.0M-NaCl | Acrylamide | 6.0 | transparent gel | crystal |
| Ex. 11 | 3.0M-NaCl | Acrylamide | 6.0 | transparent gel | crystal, precipitation mixture |
| Ex. 12 | 4.0M-NaCl | Acrylamide | 6.0 | transparent gel | precipitation |
| Ex. 13 | 1.0M-NaCl | Acrylamide | 7.0 | transparent gel | crystal |
| Ex. 14 | 2.0M-NaCl | Acrylamide | 7.0 | transparent gel | crystal |
| Ex. 15 | 3.0M-NaCl | Acrylamide | 7.0 | transparent gel | crystal, precipitation mixture |
| Ex. 16 | 4.0M-NaCl | Acrylamide | 7.0 | transparent gel | precipitation |
| Ex. 17 | 1.0M-NaCl | Acrylamide | 8.0 | transparent gel | crystal |
| Ex. 18 | 2.0M-NaCl | Acrylamide | 8.0 | transparent gel | crystal |
| Ex. 19 | 3.0M-NaCl | Acrylamide | 8.0 | transparent gel | crystal |
| Ex. 20 | 4.0M-NaCl | Acrylamide | 8.0 | transparent gel | crystal |
| Ex. 21 | 1.0M-NaCl | Acrylamide | 9.0 | transparent gel | crystal |
| Ex. 22 | 2.0M-NaCl | Acrylamide | 9.0 | transparent gel | crystal |
| Ex. 23 | 3.0M-NaCl | Acrylamide | 9.0 | transparent gel | precipitation |
| Ex. 24 | 4.0M-NaCl | Acrylamide | 9.0 | transparent gel | precipitation |
| Ex. 25 | 5%-PEG | polyethylene glycol monoacrylate | 4.0 | transparent gel | no product |
| Ex. 26 | 10%-PEG | polyethylene glycol monoacrylate | 4.0 | transparent gel | no product |
| Ex. 27 | 20%-PEG | polyethylene glycol monoacrylate | 4.0 | transparent gel | crystal |
| Ex. 28 | 30%-PEG | polyethylene glycol monoacrylate | 4.0 | transparent gel | crystal |
| Ex. 29 | 5%-PEG | polyethylene glycol monoacrylate | 5.0 | transparent gel | crystal |
| Ex. 30 | 10%-PEG | polyethylene glycol monoacrylate | 5.0 | transparent gel | crystal |
| Ex. 31 | 20%-PEG | polyethylene glycol monoacrylate | 5.0 | transparent gel | crystal |
| Ex. 32 | 30%-PEG | polyethylene glycol monoacrylate | 5.0 | transparent gel | crystal, precipitation mixture |
| Ex. 33 | 5%-PEG | polyethylene glycol monoacrylate | 6.0 | transparent gel | no product |
| Ex. 34 | 10%-PEG | polyethylene glycol monoacrylate | 6.0 | transparent gel | no product |
| Ex. 35 | 20%-PEG | polyethylene glycol monoacrylate | 6.0 | transparent gel | no product |
| Ex. 36 | 30%-PEG | polyethylene glycol monoacrylate | 6.0 | transparent gel | no product |
| Ex. 37 | 5%-PEG | polyethylene glycol monoacrylate | 7.0 | transparent gel | no product |
| Ex. 38 | 10%-PEG | polyethylene glycol monoacrylate | 7.0 | transparent gel | no product |
| Ex. 39 | 20%-PEG | polyethylene glycol monoacrylate | 7.0 | transparent gel | no product |
| Ex. 40 | 30%-PEG | polyethylene glycol monoacrylate | 7.0 | transparent gel | no product |
| Ex. 41 | 5%-PEG | polyethylene glycol monoacrylate | 8.0 | transparent gel | no product |
| Ex. 42 | 10%-PEG | polyethylene glycol monoacrylate | 8.0 | transparent gel | no product |
| Ex. 43 | 20%-PEG | polyethylene glycol monoacrylate | 8.0 | transparent gel | no product |
| Ex. 44 | 30%-PEG | polyethylene glycol monoacrylate | 8.0 | transparent gel | no product |
| Ex. 45 | 5%-PEG | polyethylene glycol monoacrylate | 9.0 | transparent gel | no product |
| Ex. 46 | 10%-PEG | polyethylene glycol monoacrylate | 9.0 | transparent gel | no product |
| Ex. 47 | 20%-PEG | polyethylene glycol monoacrylate | 9.0 | transparent gel | no product |
| Ex. 48 | 30%-PEG | polyethylene glycol monoacrylate | 9.0 | transparent gel | no product |

### <Example 49>

0.48g of sodium chloride serving as a precipitant was weighed in a 10 ml measuring flask, and the volume was adjesyted with 0.1M-citric acid buffer (pH 4.0) so as to prepare 1.2M-NaCl solution (it was used as A solution).

Moreover, into 47.5 g of 2-acrylamide 2-methylpropanesulfonic acid, was added and dissolved 2.5 g of N,N'-methylenebisacrylamide (hereunder, abbreviated MBAAm) and 50 g of deionized water, so as to prepare 50% monomer solution (it was used as B solution). Furthermore, 10% solution (it was used as C solution) of 2,2'-azobis[2-(2-imidazoline-2-yl)propane] dihydrochloride (Wako Pure Chemical Industries' VA-044) serving as the water soluble polymerization initiator was prepared. 840 µl of A solution and 160 µl of B solution were injected and mixed in a 2 ml sample tube, so as to prepare the protein crystallizing agent solution (1.0M-NaCl, pH 4.0, monomer concentration 8%). 10 µl of C solution was further added and mixed well therein, then polymerized in a warm bath at 55°C for 3 hours, to obtain a transparent hydrogel.

### <Example 50>

0.48g of sodium chloride serving as a precipitant was weighed in a 10 ml measuring flask, and the volume was adjusted with 0.1M-citric acid buffer (pH 4.0) so as to prepare 1.2M-NaCl solution (it was used as A solution).

Moreover, into 62.5 g of 80% methacryldimethylaminoethylmethyl chloride salt solution, was added and dissolved 2.5 g of N,N'-methylenebisacrylamide (hereunder, abbreviated MBAAm) and 35 g of deionized water, so as to prepare 50% monomer solution (it was used as B solution). Furthermore, 10% solution (it was used as C solution) of 2,2'-azobis[2-(2-imidazoline-2-yl)propane] dihydrochloride (Wako Pure Chemical Industries' VA-044) serving as the water soluble polymerization initiator was prepared. 840 µl of A solution and 160 µl of B solution were injected and mixed in a 2 ml sample tube, so as to prepare the protein crystallizing agent solution (1.0M-NaCl, pH 4.0, monomer concentration 8%). 10 µl of C solution was further added and mixed well therein, then polymerized in a warm bath at 55°C for 3 hours, to obtain a transparent hydrogel.

### <Example 51>

2 g of 2-methyl-2,4-pentanediol serving as a precipitant was weighed in a 10 ml measuring flask, and the volume was adjusted with 0.1M-citric acid buffer (pH 4.0) so as to prepare 20%-2-methyl-2,4-pentanediolsolution (it was used as A solution).

Moreover, into 47.5 g of dimethylacrylamide, was added and dissolved 2.5 g of N,N'-methylenebisacrylamide (hereunder, abbreviated MBAAm) and 50 g of deionized water, so as to prepare 50% monomer solution (it was used as B solution). Furthermore, 10% solution (it was used as C solution) of 2,2'-azobis[2-(2-imidazoline-2-yl) propane] dihydrochloride (Wako Pure Chemical Industries' VA-044) serving as the water soluble polymerization initiator was prepared. 840 µl of A solution and 160 µl of B solution were injected and mixed in a 2 ml sample tube, so as to prepare the protein crystallizing agent solution (1.0M-NaCl, pH 4.0, monomer concentration 8%). 10 µl of C solution was further added and mixed well therein, then polymerized in a warm bath at 55°C for 3 hours, to obtain a transparent hydrogel.

### <Example 52>

1.176 g of sodium phosphate salt and 0.035 g of potassium phosphate salt serving as precipitants were weighed in a 10 ml measuring flask, and the volume was determined by 0.1M-citric acid buffer (pH 4.0) so as to prepare 1.0M-sodium/potassium phosphate salt solution (it was used as A solution).

Moreover, into 47.5 g of 2-acrylamide 2-methylpropanesulfonic acid, was added and dissolved 2.5 g of N,N'-methylenebisacrylamide (hereunder, abbreviated MBAAm) and 50 g of deionized water, so as to prepare 50% monomer solution (it was used as B solution). Furthermore, 10% solution (it was used as C solution) of 2,2'-azobis [2-(2-imidazoline-2-yl)propane] dihydrochloride (Wako Pure Chemical Industries' VA-044) serving as the water soluble polymerization initiator was prepared. 840 µl of A solution and 160 µl of B solution were injected and mixed in a 2 ml sample tube, so as to prepare the protein crystallizing agent solution (1.0M-NaCl, pH 4.0, monomer concentration 8%). 10 µl of C solution was further added and mixed well therein, then polymerized in a warm bath at 55°C for 3 hours, to obtain a transparent hydrogel.

### <Example 53>

3.17 g of ammonium sulfate serving as a precipitant was weighed in a 10 ml measuring flask, and the volume was adjusted with 0.1M-citric acid buffer (pH 4.0) so as to prepare 2.4M-ammonium sulfate solution (it was used as A solution).

Moreover, into 62.5 g of 80% methacryldimethylaminoethylmethyl chloride salt solution, was added and dissolved 2.5 g of N,N'-methylenebisacrylamide (hereunder, abbreviated MBAAm) and 35 g of deionized water, so as to prepare 50% monomer solution (it was used as B solution). Furthermore, 10% solution (it was used as C solution) of 2,2'-azobis[2-(2-imidazoline-2-yl)propane] dihydrochloride (Wako Pure Chemical Industries' VA-044) serving as the water soluble polymerization initiator was prepared. 840 µl of A solution and 160 µl of B solution were injected and mixed in a 2 ml sample tube, so as to prepare the protein crystallizing agent solution (1.0M-NaCl, pH 4.0, monomer concentration 8%). 10 µl of C solution was further added and mixed well therein, then polymerized in a warm bath at 55°C for 3 hours, to obtain a transparent hydrogel.

### <Example 54>

1.04 g of malonic acid serving as a precipitant was weighed in a 10 ml measuring flask, and neutralized by adding 4g of 20%-sodium hydroxide solution, then the volume was adjusted with 0.1M-citric acid buffer (pH 4.0) so as to prepare 1.0M-sodium malonate solution (it was used as A solution).

Moreover, into 90g of 50% acrylamide solution (Mitsubishi Rayon's), was added and dissolved 5 g of N,N'-methylenebisacrylamide (hereunder, abbreviated MBAAm) and 5 g of deionized water, so as to prepare 50% monomer solution (it was used as B solution). Furthermore, 10% solution (it was used as C solution) of 2,2'-azobis[2-(2-imidazoline-2-yl)propane] dihydrochloride (Wako Pure Chemical Industries' VA-044) serving as the water soluble polymerization initiator was prepared. 840 µl of A solution and 160 µl of B solution were injected and mixed in a 2 ml sample tube, so as to prepare the protein crystallizing agent solution (1.0M-NaCl, pH 4.0, monomer concentration 8%). 10 µl of C solution was further added and mixed well therein, then polymerized in a warm bath at 55°C for 3 hours, to obtain a transparent hydrogel.

### INDUSTRIAL APPLICABILITY

According to the protein crystallizing device of the present invention, a protein crystallization experiment or screening of crystallization conditions can be performed rapidly and economically with a high reliability. Moreover, according to the protein crystallizing gel of the present invention, the gel reaction is completed in the protein crystallization, and furthermore a gel-like material without generating opacity can be formed, so that the transparent gel-like material holding the protein crystallizing agent can be provided.

## Claims

1. A protein crystallizing device comprising:
a protein crystallizing microarray having at least two crystallizing agent holding parts which hold a protein crystallizing agent, and
a plate laminated on said protein crystallizing microarray, said plate having
crystallizing sections corresponding to said crystallizing agent holding parts so that the sections being capable of being filled with a protein-containing sample, and
recessed parts provided between the crystallizing sections.

2. A protein crystallizing device according to claim 1, wherein said crystallizing agent holding parts are made from gels prepared in respectively different protein crystallization conditions.

3. A protein crystallizing device according to claim 1, wherein said protein crystallizing microarray is a microarray having a plurality of hollow tublar bodies in an array.

4. A protein crystallizing device according to claim 1, further comprising a mechanism which presses said protein crystallizing microarray and said plate into contact with each other.

5. A protein crystallizing device according to claim 1, wherein a sealant is filled in said recessed parts.

6. A protein crystallizing device according to claim 1, wherein a capacity of said crystallizing section is less than 0.5 µl.

7. A protein crystallizing device according to claim 1, wherein a capacity of said crystallizing section is 0.5 µl or more.

8. A protein crystallizing device according to claim 1, wherein said protein crystallizing microarray has 10 to 1000 of crystallizing agent holding parts.

9. A protein crystallizing device according to claim 1, wherein said plate further has a crystal collection mechanism which collects precipitated crystals in said crystallizing sections.

10. A protein crystallizing device according to claim 1, further comprising a detection mechanism which monitors protein crystallization in said crystallizing sections.

11. A sample filling aid for filling a protein-containing sample into said crystallizing sections of the protein crystallizing device according to any one of claims 1 through 10, comprising:
punched holes having an arrangement corresponding to said crystallizing sections, and a positioning mechanism which makes the punched holes correspond to said crystallizing sections on said plate.

12. A protein crystallizing device according to any one of claim 1 through claim 10, wherein said plate is formed with the positioning part which matches a position with a sample filling aid according to claim 11.

13. A screening method of protein crystallization conditions using the protein crystallizing device according to claim 12 and the sample filling aid according to claim 11, comprising the steps of:
placing the sample filling aid according to claim 11 on said plate so that said punched holes in the sample filling aid correspond to said crystallizing sections;
adding the protein-containing sample to said punched holes from the top of the sample filling aid so that said crystallizing sections are filled therewith;
taking out said sample filling aid ; and
laminating said plate and said protein crystallizing microarray so that said crystallizing sections and said crystallizing agent holding parts are in contact by corresponding to each other.

14. A protein crystallizing gel having sodium chloride held in a gel-like material comprising a type of monomer selected from a group consisting of acrylamide, 2-acrylamide-2-methylpropanesulfonic acid, and methacryldimethylaminoethylmethyl chloride salt.

15. A protein crystallizing gel having 2-methyl-2,4-pentanediol held in a gel-like material containing dimethylacrylamide.

16. A protein crystallizing agent having sodium/potassium phosphate held in a gel-like material containing 2-acrylamide-2-methylpropanesulfonic acid.

17. A protein crystallizing gel having ammonium sulfate held in a gel-like material containing methacryldimethylaminoethylmethyl chloride salt.

18. A protein crystallizing gel having sodium malonate held in a gel-like material containing acrylamide.

19. A protein crystallizing gel having polyethylene glycol 6000 held in a gel-like material containing polyoxyethylene monoacrylate.
